# EUROPEAN PATENT APPLICATION

(11) **EP 4 607 203 A1**
(43) Date of publication of application: **27.08.2025**
(21) Application number: 23879605.6
(22) Date of filing: 03.10.2023
(51) Int. Cl.: G01N 35/02, C12Q 1/02, C12M 1/34

(54) **DETECTION DEVICE AND METHOD FOR DETECTING TARGET MOLECULES**

(30) Priority: 20.10.2022 JP 2022168336
(71) Applicant: Toppan Holdings Inc., Tokyo 110-0016 (JP); The University of Tokyo, Bunkyo-ku, Tokyo 113-8654 (JP)
(72) Inventor: HORIUCHI, Yosuke, Tokyo 110-0016 (JP); MAKINO, Yoichi, Tokyo 110-0016 (JP); NOJI, Hiroyuki, Tokyo 113-8654 (JP)
(74) Representative: Hasegawa, Kan
(86) International application number: PCT/JP2023/036067
(87) International publication number: WO 2024/084958

(57) **Abstract**

This detection device includes a first well plate having a plurality of first wells on one surface and a second well plate having a plurality of second wells on one surface, wherein the first well plate and the second well plate are spaced apart from each other with the first wells and the second wells facing each other, and the first wells overlap the second wells in plan view.

## Description

### TECHNICAL FIELD

The present invention relates to a detection device and a method for detecting a target molecule.

Priority is claimed on Japanese Patent Application No. 2022-168336, filed on October 20, 2022, the content of which is incorporated herein by reference.

### BACKGROUND ART

Early detection of disease and prediction of the effects of medication are performed by quantitatively detecting a target molecule in a biological sample. For example, when the target molecule is a protein, quantification is performed by enzyme-linked immunosorbent assay (ELISA) or the like. In addition, when the target molecule is a nucleic acid, quantification is performed by a real-time PCR method or the like.

In recent years, there has been an increasing need for detecting a target molecule such as those described above with greater accuracy for the purpose of, for example, finding disease earlier. As a method for detecting a target molecule with high accuracy, for example, Patent Document 1, Patent Document 2, and Non Patent Document 1 describe a technique for performing an enzyme reaction in a large number of microcompartments to detect single molecules. These techniques are called digital measurement.

In digital measurement, a reaction vessel (also called a detection device) with an extremely large number of microcompartments is filled with a sample solution, so that the sample solution is dispensed into each of the microcompartments. Then, a signal from each microcompartment is binarized, and the number of target molecules is measured by determining only whether or not the target molecule contained in the sample solution is present in the microcompartment. The digital measurement can significantly improve the detection sensitivity and the quantitativeness compared with conventional methods such as ELISA, real-time PCR method and the like.

### Citation List

### Patent Documents

Patent Document 1: Japanese Patent No. 5551798
Patent Document 2: Published Japanese Translation No. 2014-503831 of the PCT International Publication

### Non Patent Document

Non Patent Document 1: Kim S. H., et al., Large-scale femtoliter droplet array for digital counting of single biomolecules., Lab on a Chip, 12 (23), 4986-4991, 2012.

### SUMMARY OF INVENTION

### Technical Problem to be Solved by Invention

In digital measurement technology, target molecules contained in a solution sample are distributed one molecule at a time to microcompartments, and the number of microcompartments to which the target molecules are distributed is measured. However, as the need for more accurate detection of target molecules is increasing as described above, there is a demand to trace back to a source of the target molecules (also called a target substance) and detect and quantify the amount of target molecules released from the target substance. For example, in digital measurement technology, when cells are the target substances and cytokines secreted from each cell are the target molecules, it is possible to detect cells (in other words, the target substances) contained in a solution sample with high accuracy, but it may be difficult to further detect and quantify the cytokines (in other words, the target molecules) secreted from each cell with high accuracy.

The present invention has been made in consideration of such circumstances, and aims to provide a detection device that can detect a target molecule released from a target substance in a solution with high sensitivity. In addition, it is also an object of the present invention to provide a method for detecting a target molecule using such a detection device.

### Solution to Problem

In order to solve the above problems, one embodiment of the present invention includes the following aspects.

[1] A detection device including a first well plate having a plurality of first wells on one surface and a second well plate having a plurality of second wells on one surface, in which the aforementioned first well plate and the aforementioned second well plate are spaced apart from each other and cause the aforementioned first wells and the aforementioned second wells to face each other, and the aforementioned first wells overlap the aforementioned second wells in plan view.
[2] The detection device according to [1], in which an opening diameter of the aforementioned second wells is smaller than an opening diameter of the aforementioned first wells, and the aforementioned first wells overlap with two or more of the aforementioned second wells in plan view.
[3] The detection device according to [1] or [2], further including a wall material which is sandwiched between the aforementioned first well plate and the aforementioned second well plate and separates the aforementioned first well plate from the aforementioned second well plate.
[4] The detection device according to [3], in which the aforementioned wall material is integrally formed with either one of the aforementioned first well plate and the aforementioned second well plate.
[5] The detection device according to [3] or [4], in which the aforementioned first well plate and the aforementioned second well plate are stacked in a freely detachable manner via the aforementioned wall material.
[6] The detection device according to any one of [1] to [5], further including a first lid material which is freely detachable from the aforementioned first well plate and forms a first member, and a second lid material which is freely detachable from the aforementioned second well plate and forms a second member, in which the aforementioned first lid material has an inlet and an outlet penetrating in the thickness direction, a flow path through which a fluid flows is formed in the aforementioned first member between the aforementioned first well plate and the aforementioned first lid material, the aforementioned second lid material has an inlet and an outlet penetrating in the thickness direction, and a flow path through which a fluid flows is formed in the aforementioned second member between the aforementioned second well plate and the aforementioned second lid material.
[7] The detection device according to any one of [1] to [6], in which a major axis of an opening of the aforementioned first well is 1 µm or more and 1 cm or less.
[8] The detection device according to any one of [1] to [7], in which an aspect ratio of the aforementioned first well is 0.002 or more and 3 or less.
[9] The detection device according to any one of [1] to [8], in which a major axis of an opening of the aforementioned second well is 1 nm or more and 1 mm or less.
[10] The detection device according to any one of [1] to [9], in which an aspect ratio of the aforementioned second well is 0.05 or more and 3 or less.
[11] The detection device according to any one of [1] to [10], in which the aforementioned first well overlaps with 1 to 10,000 of the aforementioned second wells in plan view.
[12] The detection device according to any one of [1] to [11], in which a ratio of a volume of one of the aforementioned first wells to a volume of one of the aforementioned second wells is [first well]: [second well]) = 500,000: 1 to 1: 1.
[13] A method for detecting a target molecule using the detection device according to any one of [1] to [12], the method including: a step of filling a sample solution containing a target substance into each of a plurality of the aforementioned first wells independently; a step of releasing the aforementioned target molecule from the aforementioned target substance in one of the first wells in which the aforementioned target substance is placed among a plurality of the aforementioned first wells; a step of bringing a surface onto which the aforementioned first well is formed in the aforementioned first well plate into contact with a surface onto which the aforementioned second well is formed in the aforementioned second well plate to distribute the aforementioned target molecule one molecule at a time to each of the aforementioned second wells independent of each other from the aforementioned first well; and a step of causing a reaction between the aforementioned target molecule and a detection reagent and detecting the aforementioned target molecule.
[14] The method for detecting a target molecule according to [13], wherein in the aforementioned filling step, the aforementioned sample solution is sealed in the aforementioned first well with a sealing liquid.
[15] The method for detecting a target molecule according to [14], in which the aforementioned sealing liquid is a fluorine-based oil.
[16] The method for detecting a target molecule according to any one of [13] to [15], wherein the aforementioned sample solution contains a surfactant.
[17] The method for detecting a target molecule according to any one of [13] to [16], including, prior to the aforementioned filling step, a step of obtaining the aforementioned sample solution adjusted to a concentration such that one of the aforementioned target substances is placed in each of the aforementioned first wells based on a volume of the aforementioned detection device.

### Advantageous Effects of Invention

According to the present invention, it is possible to provide a detection device that can detect a target molecule released from a target substance in a solution with high sensitivity. In addition, it is possible to provide a method for detecting a target molecule using such a detection device.

### BRIEF DESCRIPTION OF DRAWINGS

[FIG. 1] FIG. 1 is a schematic perspective view showing a detection device 100 according to a first embodiment.
[FIG. 2] FIG. 2 is a cross-sectional arrow view taken along a line segment II-II in FIG. 1.
[FIG. 3] FIG. 3 is a cross-sectional view showing another embodiment of the detection device 100.
[FIG. 4] FIG. 4 is an explanatory diagram of a method for detecting a target molecule according to the first embodiment.
[FIG. 5] FIG. 5 is an explanatory diagram of the method for detecting a target molecule according to the first embodiment.
[FIG. 6] FIG. 6 is an explanatory diagram of the method for detecting a target molecule according to the first embodiment.
[FIG. 7] FIG. 7 is an explanatory diagram of the method for detecting a target molecule according to the first embodiment.
[FIG. 8] FIG. 8 is an explanatory diagram of the method for detecting a target molecule according to the first embodiment.
[FIG. 9] FIG. 9 is an explanatory diagram of the method for detecting a target molecule according to the first embodiment.
[FIG. 10] FIG. 10 is a cross-sectional arrow view showing a detection device 200 according to the present embodiment.
[FIG. 11] FIG. 11 is an explanatory diagram of the method for detecting a target molecule according to a second embodiment.
[FIG. 12] FIG. 12 is an explanatory diagram of the method for detecting a target molecule according to the second embodiment.
[FIG. 13] FIG. 13 is an explanatory diagram of the method for detecting a target molecule according to the second embodiment.

### DESCRIPTION OF EMBODIMENTS

### [First embodiment]

Hereinafter, a first embodiment will be described in detail, with reference to the drawings as necessary. It should be noted that in the drawings, the same or corresponding parts are denoted by the same or corresponding reference numerals, and duplicated explanations will be omitted. Some of the dimensional ratios in each drawing are exaggerated for the sake of illustration, and do not necessarily match the actual dimensional ratios.

### [Detection device]

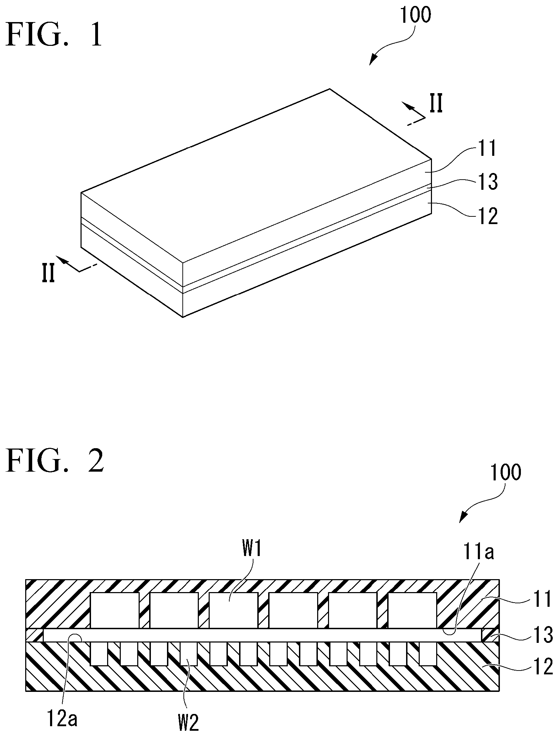

FIG. 1 is a schematic perspective view showing a detection device 100 of the present embodiment. FIG. 2 is a cross-sectional arrow view taken along a line segment II-II in FIG. 1.

The detection device 100 has a first well plate 11, a second well plate 12, and a wall material 13. The detection device 100 is used as a reaction vessel for accommodating a sample inside, releasing a target molecule from a target substance contained in the sample, and carrying out a detection reaction of the target molecule. At that time, the target molecule is released from the target substance contained in the sample in the first well plate 11, and the target molecule moving from the first well plate is detected in the second well plate 12.

The first well plate 11 and the second well plate 12 are stacked in a freely detachable manner via the wall material 13.

### <First well plate, second well plate>

The first well plate 11 is a plate-like member that has a rectangular shape in plan view. A "plan view" refers to a field of view from the normal direction of the upper surface of the first well plate 11.

The first well plate 11 has a plurality of first wells W1 on a well-forming surface 11a that faces the inner surface of the detection device 100. The first wells W1 are recesses provided on the well-forming surface 11a of the first well plate 11 and open to the well-forming surface 11a.

The second well plate 12 is a plate-like member that has a rectangular shape in plan view, and has the same contour shape as that of the first well plate 11 in plan view. The first well plate 11 and the second well plate 12 are arranged so that the first wells W1 and the second wells W2 face each other, and are spaced apart from each other.

The second well plate 12 has a plurality of second wells W2 on a well-forming surface 12a that faces the inner surface of the detection device 100. The second wells W2 are recesses provided on the well-forming surface 12a of the second well plate 12 and open to the well-forming surface 12a.

The materials of the first well plate 11 and the second well plate 12 may or may not have electromagnetic wave transmissivity. Here, examples of electromagnetic waves that are the subject of the transmissivity judgment include X-rays, ultraviolet rays, visible light, and infrared rays. When the first well plate 11 and the second well plate have electromagnetic wave transmissivity, it becomes possible to use electromagnetic waves in order to analyze the results of experiments performed with the detection device 100. For example, the fluorescence or phosphorescence generated as a result of irradiation with electromagnetic waves can be measured through the first well plate 11 or the second well plate. It should be noted that the term "electromagnetic wave transmissivity" refers to the property of being able to transmit electromagnetic waves of various wavelengths, such as radio waves, light, X-rays, and gamma rays.

Examples of the materials having electromagnetic wave transmissivity include glass and resins. Examples of the resins include ABS resins, polycarbonate, COC (cycloolefin copolymers), COP (cycloolefin polymers), acrylic resins, polyvinyl chloride, polystyrene, polyethylene, polypropylene, polyvinyl acetate, PET (polyethylene terephthalate), PEN (polyethylene naphthalate), PC (polycarbonate), silicone resins, fluororesins, and amorphous fluororesins. These resins may contain various additives, or may be polymer alloys in which a plurality of resins are mixed.

The materials having electromagnetic wave transmissivity preferably have substantially no autofluorescence. The phrase "have substantially no autofluorescence" means that the material does not have autofluorescence at all at the wavelength used for sample detection, or if it does, it is so weak that it does not affect sample detection. For example, if the autofluorescence is about equal to or less than 1/2 or equal to or less than 1/10 of the fluorescence of an object to be detected, it can be said to be so weak that it does not affect the sample detection. When the first well plate 11 and the second well plate 12 are formed using such a material, the detection sensitivity can be increased in sample detection using electromagnetic waves.

Examples of the materials that have electromagnetic wave transmissivity and do not emit autofluorescence include quartz glass. Examples of the materials that have weak autofluorescence and do not interfere with sample detection using electromagnetic waves include low fluorescence glass, acrylic resins, COC (cycloolefin copolymers), and COP (cycloolefin polymers).

The material of the first well plate 11 is preferably softer (also referred to as having a lower elastic modulus) than the second well plate 12. Of the above-mentioned materials, for example, a silicone resin is preferred as the material of the first well plate 11. As the silicone resin, for example, PDMS (polydimethylsiloxane) can be suitably used.

The thickness of the second well plate 12 can be appropriately determined. When observing the fluorescence from the second well plate 12 side using a fluorescence microscope, the thickness of the second well plate 12 may be, for example, 5 mm or less, 2 mm or less, or 1.6 mm or less. The thickness of the first well plate 11 can be appropriately determined within a range that does not impair the effects of the invention.

The first well plate 11 and the second well plate 12 may be constituted of a single layer using only the above-mentioned material, or may be a laminate of a plurality of materials. When the laminate is processed to form the first well plate 11 and the second well plate 12, a layer having the well (the first well W1 or the second well W2) and a layer supporting the former layer may be made of different materials. For example, a laminate in which a fluororesin is laminated on a substrate may be used as the material of the first well plate 11, and a well array may be formed by processing a layer of this fluororesin. The same applies to the second well plate 12. For example, CYTOP (registered trademark) (AGC Inc.) or the like can be used as the fluororesin.

### (First well, second well)

The first well W1 accommodates the sample contained inside the detection device 100 and is used as a place to release the target molecule from the target substance contained in the sample.

Further, the second well W2 accommodates the target molecule released in the first well W1 and functions as a reaction site between the target substance and a reagent for detecting the target substance.

### (Well shape)

Various shapes can be adopted for the shape of the first well W1 and the second well W2. Examples thereof include cylindrical shapes such as a cylinder, an elliptic cylinder, and a polygonal cylinder, pyramidal shapes such as a cone and a pyramid, and truncated pyramidal shapes such as a truncated cone and a truncated pyramid. When the first well W1 and the second well W2 have pyramidal or truncated pyramidal shapes, the shapes are preferably such that the opening diameter gradually decreases in the depth direction of the well.

Bottom portions of the first well W1 and the second well W2 may be flat or may have a curved surface (convex surface or concave surface).

It should be noted that the expression "the same shape and the same size" may be satisfied as long as the shapes are the same and the volumes are the same to the extent required for the digital measurement using the detection device 100, and a variation approximately within a manufacturing error may be accepted.

A major axis of an opening of the first well W1 is preferably 1 µm or more and 1 cm or less. It should be noted that the term "major axis" refers to the maximum distance between two parallel lines circumscribing the opening of the first well W1 when seen in plan view.

When the shape of the first well W1 in plan view is circular, the diameter of the opening of the first well W1 (also called the opening diameter) may be, for example, about 10 to 500 µm. Further, a depth of the first well W1 may be, for example, about 5 to 500 µm. The "depth of the first well W1" refers to a distance from an "imaginary plane parallel to the well-forming surface 11a and in contact with the well-forming surface 11a" to a "deepest part of the first well W1".

The major axis of the opening of the first well W1 is preferably 1 nm or more and 1 mm or less. It should be noted that the term "major axis" refers to the maximum distance between two parallel lines circumscribing the opening of the first well W1 when seen in plan view.

When the shape of the second well W2 in plan view is circular, the diameter of the opening of the second well W2 (opening diameter) may be, for example, about 1 to 100 µm. Further, a depth of the second well W2 may be, for example, about 1 to 100 µm. The "depth of the second well W2" refers to a distance from an "imaginary plane parallel to the well-forming surface 12a and in contact with the well-forming surface 12a" to a "deepest part of the second well W2".

An aspect ratio of the first well W1 is preferably 0.002 or more and 3 or less. It should be noted that the "aspect ratio of the first well W1" refers to a ratio between the depth of the first well W1 and the major axis of the first well W1 ([depth of the first well W1]/ [major axis of the first well W1]).

An aspect ratio of the second well W2 is preferably 0.05 or more and 3 or less. It should be noted that the "aspect ratio of the second well W2" refers to a ratio between the depth of the second well W2 and the major axis of the second well W2 ([depth of the second well W2]/[major axis of the second well W2]).

### (Well volume)

A volume of one first well W1 may be about 1,000 fL (1 pL) to 100 nL. In the detection device 100, the plurality of first wells W1 may have the same shape and the same size, or the size and shape of each first well W1 may be different.

Further, a volume of one second well W2 may be about 1 fL to 1,000 pL (1 nL). In the detection device 100, the plurality of second wells W2 may have the same shape and the same size, or the size and shape of each second well W2 may be different.

A ratio between the volume of one first well W1 and the volume of one second well W2 [volume of the first well W1]: [volume of the second well W2] is preferably from 500,000:1 to 1:1, that is, within a range of 500,000:1 and 1:1.

### (Number of wells)

The detection device 100 has 10 to 10,000 first wells W1. Further, in the detection device 100, the first wells W1 are arranged so as to overlap the second wells W2 in plan view. In this case, the first well W1 may be configured so as to overlap with the second well W2 in a 1:1 ratio in plan view, or may be configured so as to overlap with two or more second wells W2 in plan view.

The number of second wells W2 that overlap with one first well W1 in plan view is from 1 to 10,000, and preferably from 10 to 10,000. In other words, the second well plate 12 may be formed with second wells W2 of a size and number that satisfy the above number.

### (Arrangement of wells)

The arrangement of the first wells W1 in the first well plate 11 and the second wells W2 in the second well plate 12 is not particularly limited. The plurality of first and second wells may be arranged, for example, in a triangular lattice pattern, a square lattice pattern, or irregularly arranged.

The first well plate 11 and the second well plate 12 described above can be produced using known injection molding, microimprinting technology, or nanoimprinting technology. Further, the first well plate 11 and the second well plate 12 can also be produced manufactured by forming wells by etching using known photolithography technology.

### <Wall material>

The wall material 13 is formed in a closed ring shape in plan view and is sandwiched between the first well plate 11 and the second well plate 12. The wall material 13 functions as a spacer that separates the first well plate 11 and the second well plate 12 and creates a space between the first well plate 11 and the second well plate 12.

As the material of the wall material 13, the same material as that of the first well plate 11 and the second well plate 12 described above can be employed. The wall material 13 formed of such a material can be integrated with the first well plate 11 or the second well plate 12 by adhesion with an adhesive or by welding through thermal welding, ultrasonic welding, laser welding, or the like.

Further, the material of the wall material 13 may be different from that of the first well plate 11 and the second well plate 12. The wall material 13 may be, for example, a double sided tape or an adhesive. A base material of the double sided tape may be a plastic or a paper material. The adhesive may be grease or a fast curing adhesive containing a component such as α-cyanoacrylate.

It should be noted that the wall material 13 may be formed simultaneously (in other words, integrally) with the production of either one of the first well plate 11 and the second well plate 12, and may be integrated with the first well plate 11 or the second well plate 12.

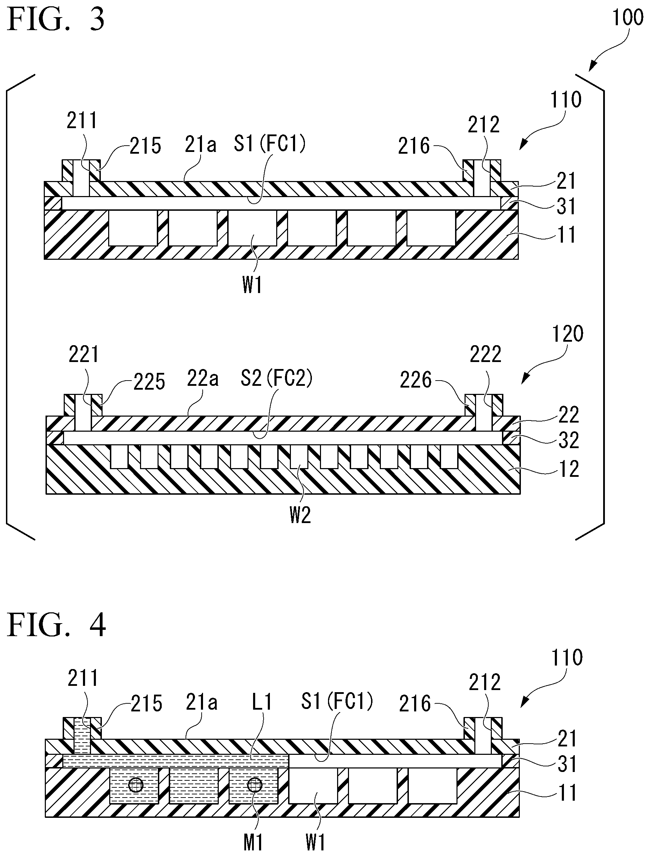

When using the detection device 100, the first well plate 11 and the second well plate 12 are separated and used. FIG. 3 is a cross-sectional view showing another embodiment of the detection device 100. The detection device 100 is used by separating it into a first member 110 and a second member 120.

The first member 110 has a first well plate 11, a first lid material 21 that is freely detachable from the first well plate 11, and a first wall material 31 sandwiched between the first well plate 11 and the first lid material 21.

The first lid material 21 has the same contour shape as that of the first well plate 11 in plan view.

The first lid material 21 has two through holes that penetrate in the thickness direction. The two through holes are provided at one end side and the other end side of the first lid material 21, respectively. One of the through holes is an inlet 211 used when injecting a liquid product into an internal space S1 of the first member 110, and the other through hole is an outlet 212 used when discharging a liquid product from the internal space S1.

Here, the above "liquid product" includes not only a liquid sample, but also a detection reagent and a sealing liquid.

The inlet 211, the internal space S1, and the outlet 212 are connected in this order and forming a flow path FC1 as a whole. In the first member 110, the liquid product is appropriately caused to flow through the flow path FC1 to seal the target substance in the first well W1. In plan view, a plurality of first wells W1 are arranged between the inlet 211 and the outlet 212.

A cylindrical injection port 215 surrounding the periphery of the inlet 211 is provided on an upper surface 21a of the first lid material 21. The injection port 215 is connected to the inlet 211. The injection port 215 is used to connect a syringe, for example, when filling the internal space with a liquid product using a syringe filled with the liquid product.

Similarly, a cylindrical discharge port 216 surrounding the periphery of the outlet 212 is provided on the upper surface 21a of the first lid material 21. The discharge port 216 is connected to the outlet 212. The discharge port 216 is used to connect a tube through which a liquid product flows, for example, when extracting the liquid product from the internal space S1.

Further, the second member 120 has a second well plate 12, a second lid material 22 that is freely detachable from the second well plate 12, and a second wall material 32 sandwiched between the second well plate 12 and the second lid material 22.

The second lid material 22 has the same contour shape as that of the second well plate 12 in plan view.

The second lid material 22 has an inlet 221 and an outlet 222 that penetrate in the thickness direction. The inlet 221, an internal space S2 of the second member 120, and the outlet 222 are connected in this order and forming a flow path FC2 as a whole. In the second member 120, a liquid buffer is caused to flow through the flow path FC2 and sealed in the second well W2. In plan view, a plurality of second wells W2 are arranged between the inlet 221 and the outlet 222.

The upper surface 22a of the second lid material 22 is provided with a cylindrical injection port 225 that surrounds the periphery of the inlet 221 and communicates with the inlet 221. In addition, the upper surface 22a is provided with a cylindrical discharge port 226 that surrounds the periphery of the outlet 212 and communicates with the outlet 212.

As the material of the first lid material 21 and the second lid material 22, the materials exemplified above as the materials of the first well plate 11 and the second well plate 12 can be employed. The first lid material 21 and the second lid material 22 can be produced by a known injection molding technique.

### [Method for detecting target molecule]

FIGS. 4 to 9 are explanatory diagrams of the method for detecting a target molecule in the present embodiment. The method for detecting a target molecule includes steps of performing the following operations. In the method for detecting a target molecule, the detection device 100 described above is used to detect a target molecule contained in a sample in the second well W2 of the detection device 100.
(1) A step of adjusting the concentration of a liquid sample containing a target substance to obtain a sample solution
(2) A step of filling the sample solution into each of a plurality of first wells independently
(3) A step of releasing a target molecule from the target substance in the first well
(4) A step of distributing one target molecule at a time per each of the second wells
(5) A step of detecting the target molecule

### (1) A step of adjusting the concentration of a liquid sample containing a target substance to obtain a sample solution

First, the concentration of a liquid sample containing a target substance is adjusted to obtain a sample solution L1. Hereinafter, this step may be abbreviated as "step (1)".

The sample includes, for example, a biological sample or an environmental sample. The biological sample is not particularly limited, and examples thereof include serum, plasma, urine, and cell culture media. Further, examples of the environmental sample include river water and industrial wastewater.

The biological sample and the environmental sample contain a target substance that releases a target molecule to be detected. Examples of the target substance include DNA, RNA, proteins, viruses, cells, and specific compounds. Examples of RNA include miRNA and mRNA. Further, examples of cells include bacteria, yeast, animal cells, plant cells, and insect cells.

The sample may contain a reaction reagent for detecting the target substance. Examples of the reaction reagent include a buffer substance, an enzyme, a substrate, an antibody, and an antibody fragment. For example, when the target substance is a nucleic acid, in order to carry out a biochemical reaction such as an enzyme reaction with a template nucleic acid related to the target substance, the enzyme is selected according to the content of the biochemical reaction. The biochemical reaction with the template nucleic acid is, for example, a reaction that generates a signal amplification under conditions in which the template nucleic acid is present. In addition, when detecting a protein, it may be detected through an enzyme reaction by binding an enzyme to an antibody. HRP, alkaline phosphatase, β-galactosidase or the like may be used as a detection enzyme.

The reaction reagent is selected according to the detection reaction to be adopted. Specific examples of the detection reaction include an invasive cleavage assay (ICA) method, a loop-mediated isothermal amplification (LAMP) method (registered trademark), a 5'→3' nuclease method (TaqMan (registered trademark) method), and a fluorescent probe method.

The sample may contain a surfactant. Examples of the surfactant include Triton-X100 (also called polyethylene glycol mono-4-octylphenyl ether (n = approximately 10)), sodium dodecyl sulfate, Nonidet P-40 (also called octylphenoxy poly(ethyleneoxy)ethanol), and Tween 20 (also called polyoxyethylene sorbitan monolaurate).

The concentration of the surfactant is preferably 0.0011 g/L or more and 55.5 g/L or less (0.0001 to 5% by volume [v/v, volume percentage concentration]), more preferably 0.0111 g/L or more and 22.2 g/L or less (0.001 to 2% by volume), and still more preferably 0.111 g/L or more and 11.1 g/L or less (0.01 to 1% by volume) with respect to the total volume of the sample.

If the concentration of the surfactant is 55.5 g/L or less with respect to the total volume of the sample, there are less adverse effects on the reaction in the subsequent detection of the target substance.

The concentration of the sample is adjusted to a concentration at which one target substance M1 is placed in each first well W1 based on the volume of the detection device 100 used. The concentration of the sample is preferably determined by conducting a preliminary experiment using the detection device 100.

### (2) A step of filling the sample solution into each of a plurality of first wells independently

Subsequently, each of a plurality of the first wells W1 is filled with the sample solution L1 independently. Hereinafter, this step may be abbreviated as "step (2)". The detection device 100 is divided into a first member 110 and a second member 120, and the first member 110 is used in this step.

First, as shown in FIG. 4, the sample solution L1 containing the detection reagent is injected into the internal space S1 from the inlet 211. When the sample solution L1 flows through the internal space S1 (flow path FC1), the first wells W1 that are opened to the internal space S1 are filled with the sample solution L1.

After the internal space S1 and all the first wells W1 are filled with the sample solution L1, the sample solution L1 that has passed through the flow path FC1 is discharged from the outlet 212.

At this time, by adjusting the concentration of the sample solution L1 in advance, one target substance M1 is filled into one first well W1. More specifically, when the volume of the internal space S1 (that is, the volume of the sample solution L1 injected into the internal space S1) and the total number of first wells W1 are already known, the concentration of the sample solution L1 is adjusted so that the number of target substances M1 injected into the internal space S1 is, for example, 1/10 or less of the total number of first wells W1. With this operation, one first well W1 is filled with one or less, that is, 0 or 1 target substance M1.

The means for introducing the target substance M1 into the first well W1 is not particularly limited, and an appropriate means can be selected in accordance with the target substance M1 to be detected. For example, the target substance M1 may be distributed to the first well W1 by its own weight, or may be introduced into the first well W1 by distribution through diffusion.

Further, a substance that captures the target substance M1 (also called a capture substance) may be used, and the target substance M1 that does not easily settle by its own weight may be delivered by causing the capture substance to bound thereto. Furthermore, the efficiency of introducing the target substance M1 into the first well W1 can also be improved by fixing the capture substance to the first well W1 in advance and allowing the capture substance to capture the target substance M1 delivered together with the sample solution.

Alternatively, the capture substance may be brought into contact with the target substance M1 in the first well W1 by introducing the target substance M1 into the first well W1 after introducing the capture substance into the first well W1.

The capture substance is a substance that can capture the target substance M1. The capture substance may be, for example, a conjugate of a solid phase and a specific binding substance with respect to the target substance M1.

Examples of the specific binding substance include an antibody, an antibody fragment, and an aptamer. Examples of the antibody fragment include Fab, F(ab')2, Fab', a single chain antibody (scFv), a disulfide-stabilized antibody (dsFv), a dimeric V-region fragment (Diabody), and a peptide including CDR. The antibody may be a monoclonal antibody or a polyclonal antibody. It may also be a commercially available antibody.

Further, when the target substance M1 includes a sugar chain, the specific binding substance may be a lectin. Moreover, when the target substance M1 includes a lipid membrane, the specific binding substance may be a substance that has binding properties to lipid membranes. Examples of the substance that has binding properties to lipid membranes include a hydrocarbon such as hexanediol and a membrane protein such as a transmembrane protein. Examples of the membrane protein include α-hemolysin.

Examples of the solid phase that constitutes the capture substance include particles, films, and substrates. Further, the specific binding substance with respect to the target substance M1 may be of one type, or may be two or more types. For example, there may be three types, four types, or five or more types.

The particles are not particularly limited, and examples thereof include polymer particles, magnetic particles, and glass particles. The particles are preferably subjected to a surface treatment in order to avoid nonspecific adsorption. Further, in order to fix the specific binding substance, particles having a functional group such as a carboxyl group on the surface are preferred. More specifically, a product named "Magnosphere LC300" manufactured by JSR Corporation and the like can be used.

The method of fixing the specific binding substance on the solid phase is not particularly limited, and examples thereof include a method based on physical adsorption, a method based on chemical bonding, a method of using avidin-biotin binding, and a method of using binding between protein G or protein A and an antibody.

As the method by physical adsorption, there is a method of fixing a specific binding substance on the particle surface by hydrophobic interaction or electrostatic interaction.

As the method by chemical bonding, there is a method using a crosslinking agent. For example, in the case where the surface of a particle has a hydroxyl group, the specific binding substance can be fixed on the particle surface by reacting a crosslinking agent with the carboxyl group of the specific binding substance to form an active ester, and then reacting the hydroxyl group with this ester group.

It is preferable to provide a spacer between the specific binding substance and the particle surface so as not to inhibit the ability of the specific binding substance for recognizing the target substance M1.

In addition, for example, when a virus is used as the target substance M1, a cell to which the virus can attach (that is, a cell having a virus receptor) may be used as the capture substance.

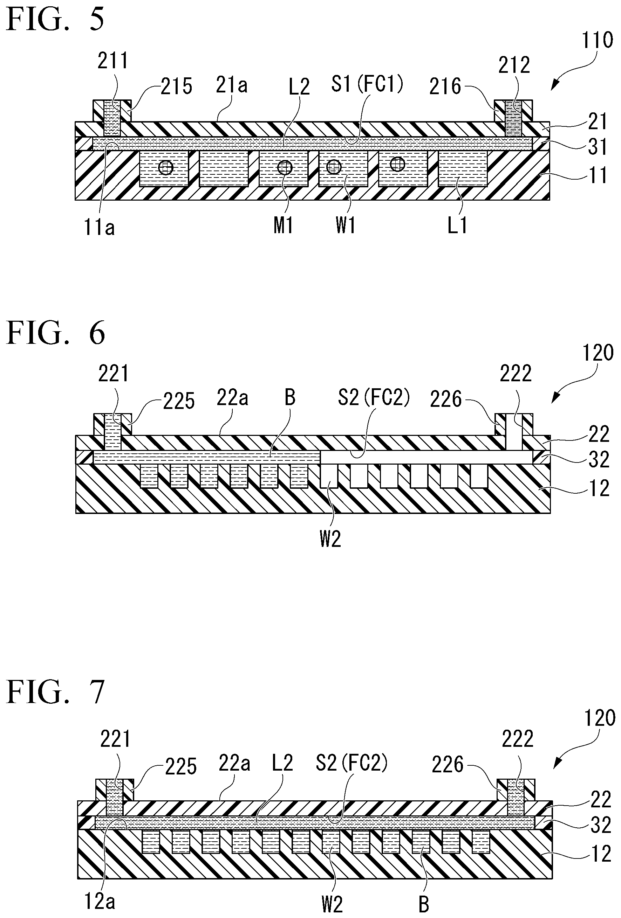

Subsequently, as shown in FIG. 5, a sealing liquid L2 is injected into the internal space S1 from the inlet 211. The sealing liquid L2 flows in the internal space S1 in the surface direction of the well-forming surface 11a, and replaces the internal space S1 by washing away the sample solution L1 that is not contained in the first well W1 among the sample solution L1 delivered to the flow path FC1.

The sealing liquid L2 is preferably an oily solution, and more preferably an oil. As the oil, fluorine-based oil, silicone-based oil, hydrocarbon-based oil, a mixture thereof or the like can be used.

More specifically, a product named "FC-40" (CAS number: 86508-42-1) manufactured by Sigma-Aldrich Co., LLC or the like can be used. FC-40 is a fluorinated aliphatic compound (fluorine-based oil) and has a specific gravity of 1.85 g/mL at 25°C.

As a result, the sealing liquid L2 seals each of the plurality of first wells W1 individually, and the first wells W1 containing the sample solution L1 become independent reaction spaces. The sample solution L1 replaced by the sealing liquid L2 and the excess sealing liquid L2 are discharged from the outlet 212.

### (3) A step of releasing a target molecule from the target substance in the first well

Subsequently, the target molecule is released from the target substance M1 distributed to each first well W1. Hereinafter, this step may be abbreviated as "step (3)".

The method of releasing the target molecule from the target substance is selected in accordance with the type of the target substance of interest. For example, when the target substance is DNA or RNA, a plurality of fragments (that is, target molecules) may be obtained from one fragment by ultrasonic disintegration or enzymatic degradation.

When the target substance is an aggregate formed by hybridization between nucleic acids, the detection device 100 may be heated and each nucleic acid may be separated by thermal denaturation.

When the target substance is a protein aggregate, the aggregate may be loosened by ultrasonic irradiation, an enzyme that degrades the protein may be used, or a reagent that breaks down the protein aggregate may be added.

When the target substance is a cell, the cell may be disrupted by ultrasonic waves, or the cell membrane may be disrupted by applying electrical stimulation.

Examples of specific methods include the following method.

First, beads are placed in the first well W1. The beads may be placed in the first well W1 in advance, or may be added to a solution containing the cells and placed in the first well W1 together with the cells. The beads are not limited as long as they do not impair the effects of the invention and have a size that allows them to be placed in the first well W1. The material of the beads may be silica or a magnetic material.

The size of the beads is preferably from 50 nm to 500 µm, more preferably from 100 nm to 100 µm, and still more preferably from 500 nm to 10 µm.

At least 10 or more beads are preferably placed in the first well W1.

Next, while both the cells and the beads as the target substances are in the first wells W1, the first wells W1 are made independent of each other by step (2) above and the cells are cultured. After culturing the cells for a certain period of time, ultrasonic waves are irradiated to the detection device 100. As a result, it is possible to disrupt the cell walls of the cells by causing the beads to vibrate in the first well W1, and to release the contents of the cells into the first well W1.

Further, when the target substance M1 is a cell, the cell membrane may be dissolved using a reagent that dissolves the cell membrane.

Examples of specific methods include the following method.

First, a solution containing cells is mixed with a reagent that dissolves the cell membrane (also called a cell lysis solution). The solution containing cells and the cell lysis solution may be mixed before being injected into the detection device 100, or may be mixed in the detection device 100.

When mixing the solution containing cells and the cell lysis solution in the detection device 100, there is a method of first injecting the solution containing cells into the first well W1, and then injecting the cell lysis solution into the first well W1. Possible methods for adding the cell lysis solution to the first well W1 include adding it to the detection device 100 at a flow rate that does not cause the cells placed in the first well W1 to flow out of the first well W1, or fixing the cells in the first well W1.

The method of fixing the cells in the first well W1 may be a method of binding a cell capture substance to the first well W1, or may be a method of placing particles to which a capture substance is bound in the first well W1. Examples of the "capture substance" include an antibody that binds to the cells. When using particles to which a capture substance is bound, it is preferable to use magnetic beads as the particles, and to retain the particles in the first well W1 by magnetic force when a cell lysis solution is added to the first well W1.

A surfactant, a commercially available reagent or the like can be used as the cell lysis solution.

After the cells and the cell lysis solution are added into the first wells W1, the cell membrane is dissolved by making the first wells W1 independent of each other by step (2) and allowing the reaction to proceed for a certain period of time. As a result, the contents of the cells can be released into the first well W1.

Further, it is also possible to culture cells as the target substance for a certain period of time in the first well W1, and detect secretion products such as cytokines secreted from the cells as the target molecules.

### (4) A step of distributing one target molecule to each second well

Subsequently, the target molecules released into the sample solution L1 are distributed, one molecule per each second well W2.

First, as shown in FIG. 6, a liquid buffer B is injected into the internal space S2 from the inlet 221 of the second member 120. It is preferable that the buffer B contain a detection reagent for the target molecule. When the buffer B flows through the internal space S2 (that is, the flow path FC2), the buffer B fills the second well W2 that opens into the internal space S2.

After the internal space S2 and all the second wells W2 are filled with the buffer B, the buffer B that has passed through the flow path FC2 is discharged from the outlet 222.

Subsequently, as shown in FIG. 7, the sealing liquid L2 is injected into the internal space S2 from the inlet 221. The sealing liquid L2 flows in the internal space S1 in the surface direction of the well-forming surface 12a, and replaces the internal space S2 by washing away the buffer B that is not contained in the second well W2 among the buffer B delivered to the flow path FC2.

The same sealing liquid as that used in the above step (2) can be used as the sealing liquid L2. The sealing liquid used in step (2) and the sealing liquid used in this step may be the same or different.

As a result, the sealing liquid L2 seals each of the plurality of second wells W2 individually, and the second wells W2 containing buffer B become independent. The buffer B replaced by the sealing liquid L2 and the excess sealing liquid L2 are discharged from the outlet 222.

Then, the first lid material 21 is removed from the first member 110 to form the first well plate 11. Further, the second lid material 22 is removed from the second member 120 to form the second well plate 12. The well-forming surfaces 11a, 12a of these well plates are covered with the sealing liquid L2.

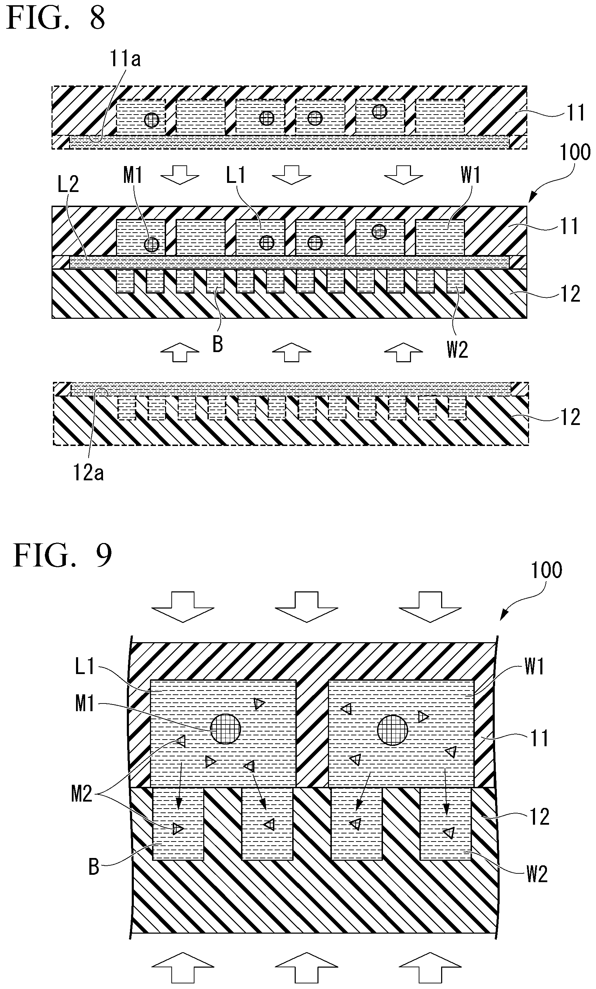

Then, as shown in FIG. 8, the well-forming surface 11a of the first well plate 11 and the well-forming surface 12a of the second well plate 12 are superimposed so as to face each other to form the detection device 100. At this time, the first well W1 is placed so as to overlap the second well W2 in plan view.

Furthermore, in the detection device 100, the first well plate 11 and the second well plate 12 are pressed against each other so as to bring the well-forming surface 11a into contact with the well-forming surface 12a. When the first well plate 11 and the second well plate 12 are pressed against each other, an experimenter may directly press them manually, or may press them using an instrument such as a clip or a roller. Further, the pressure applied to press the first well plate 11 and the second well plate 12 may be the force of the experimenter himself, or may be that of a weight.

As a result, as shown in FIG. 9, the first well W1 and the second well W2 are connected, and a target molecule M2 in the first well W1 diffuses and moves to the second well W2.

The target molecule M2 moved to the second well W2 may be dissolved in the sample solution L1 in the second well W2, or may be captured by a target molecule capture substance fixed to the second well W2. The target molecule capture substance is a substance having a functional group that specifically binds to the target molecule M2, and may be, for example, an antibody if the target molecule M2 is a protein, or a nucleic acid that forms a complementary strand if the target molecule M2 is a nucleic acid.

Further, in a state where a particle having a target molecule capture substance (hereinafter, referred to as a capture particle) is placed in the first well W1, the target molecule M2 may be released from the target substance M1 in the first well W1, and the released target molecule M2 may be captured by the capture particle. The target molecule M2 can be introduced into the second well W2 by causing the capture particle that has captured the target molecule M2 to move from the first well W1 to the second well W2.

When using a capture particle as described above, the capture particle may be moved from the first well W1 to the second well W2 by causing the capture particle to settle or float using the specific gravity difference between the sample solution L1 and the capture particle. Further, when a magnetic bead is used as the capture particle, the behavior of the capture particle may be controlled by using a magnetic force from the outside of the detection device 100.

### (5) A step of detecting the target molecule

Then, the target molecule M2 is caused to react with the detection reagent to detect the target molecule M2.

Examples of the detection method include a method involving a signal amplification reaction inside the sealed second well W2. Inside the second well W2, a signal derived from the detection reagent is amplified so that the signal is detected.

Examples of the signal include fluorescence, color development, potential change, and pH change.

The signal amplification reaction may be, for example, a biochemical reaction, and more specifically, an enzyme reaction. As an example, the signal amplification reaction is an isothermal reaction in which while a reagent solution containing an enzyme for signal amplification is contained in the second well W2, the detection device 100 is maintained under a constant temperature condition at which a desired enzyme activity is obtained, for example, at 60°C or higher, and preferably about 66°C, for a predetermined period of time, for example, at least 10 minutes, and preferably about 15 minutes.

Specific examples of the signal amplification reaction include, when the target molecule M2 is a nucleic acid, an ICA reaction such as an Invader (registered trademark) method, a loop-mediated isothermal amplification (LAMP (registered trademark) method), a 5'→3' nuclease method (TaqMan (registered trademark) method), and a fluorescent probe method. It is thought that these methods can cause a signal amplification reaction even if the above-mentioned surfactant (which may be contained in the reagent or cell lysis solution) is contained in the solution.

Further, when the cell lysis solution inhibits the reaction during the signal amplification reaction, the solution in the second well W2 may be replaced. In that case, a detection reagent may be added from the outside while fixing the target molecule M2 in the second well W2.

As a method of fixing the target molecule in the second well W2, a substance that captures the target molecule M2 may be bound to the second well W2, or magnetic particles to which a compound that captures the target molecule M2 is bound may be added to the second well W2 in advance and the magnetic particles may be fixed by magnetic force.

As the signal amplification reaction, it is particularly preferable to use the ICA reaction. In the ICA reaction, signal amplification proceeds through a cycle of two reactions: (1) complementary binding between nucleic acids; and (2) recognition and cleavage of a triple-stranded structure by an enzyme. In such a signal amplification reaction, the effects of reaction cycle inhibition by impurities other than the target molecule are small. Therefore, even if various components (impurities) other than the target molecule are present in the second well W2, the target molecule can be detected with high accuracy by using the ICA reaction.

For example, when an ICA reaction is used for the signal amplification reaction, the sample solution L1 contains a reaction reagent and a template nucleic acid required for the ICA reaction. When the biochemical reaction in the reaction step is an ICA reaction, in the second well W2 where the target molecule is present, a fluorescent substance is released from a quenching substance, thereby emitting a predetermined fluorescence signal in response to the excitation light.

Further, the detection of a target molecule can also be performed by binding a substance that specifically binds to the target molecule (specific binding substance) to the target molecule and detecting the specific binding substance that has been bound. For example, when the target molecule is a protein, the ELISA method can be used for detection. More specifically, the detection may also be performed by, for example, sandwich ELISA using the principle of FRET.

When performing the sandwich method using the principle of FRET, first, a first specific binding substance (for example, an antibody) labeled with a first fluorescent substance (donor) and a second specific binding substance labeled with a second fluorescent substance (acceptor) that has an absorption wavelength that overlaps with a fluorescence wavelength of the first fluorescent substance are prepared.

Then, a target molecule (for example, an antigen) is brought into contact with both the first specific binding substance and the second specific binding substance to form a complex. When a complex is formed, a distance between the donor and the acceptor becomes short, and the fluorescence wavelength of the acceptor can be detected by irradiation with the excitation wavelength of the donor. Alternatively, the specific binding substance may be labeled with a nucleic acid fragment, and then the nucleic acid fragment may be detected by the ICA reaction.

As the specific binding substance, those similar to the specific binding molecule for the structure, which will be described later, such as an antibody, an antibody fragment, and an aptamer can be used. In order to detect a specific binding substance bound to the target molecule, the specific binding substance may be directly or indirectly labeled with, for example, an enzyme such as horseradish peroxidase (HRP). When two or more specific binding substances are used, each of the specific binding molecules can be labeled in a manner that makes them distinguishable from each other.

A signal observation method can be selected from known appropriate methods depending on the type of the signal to be observed. For example, when performing bright field observation, white light is irradiated in a vertical direction onto a substrate on which a well array is provided. When observing a fluorescence signal, excitation light corresponding to the fluorescent substance is irradiated into the well from the bottom side of the well, and the fluorescence emitted by the fluorescent substance is observed. An image of the entirety or a part of the observed well array is captured and saved, and image processing is performed by a computer system.

According to the detection device 100 configured as described above, it becomes possible to detect a target molecule released from a target substance in a solution with high sensitivity.

In addition, according to the method for detecting a target molecule as described above, it is possible to analyze a target molecule released from one target substance. Furthermore, the characteristics of the target substance that released the target molecule after the detection of the target molecule can be analyzed, for example, by analyzing the DNA or RNA of cells after detecting cytokines, or by counting the number of specific DNA sequences and then analyzing the entire sequence of the DNA. These make it possible to detect the target molecule with high accuracy.

It should be noted that although step (1) is performed in the present embodiment, step (1) may be omitted. When step (1) is not performed, after filling the first well with the sample solution, it is preferable to extract the first well W1 in which one target substance M1 is placed from among a plurality of first wells in step (3), and to perform subsequent steps (4) and (5).

At that time, by visualizing the target substance M1, it becomes easier to extract the first well in which one target substance M1 is placed. When the target substance M1 is a cell, a possible visualization method is to bring the cell into contact with a staining reagent in advance.

Further, when the target substance M1 is a nucleic acid, a possible visualization method is to mix the target substance M1 with a known DNA staining reagent before being injected into the detection device 100. After staining, only the nucleic acid may be recovered by a known method, and the recovered nucleic acid may be added to the sample solution L1 again, injected into the detection device 100, and placed in the first well W1.

In addition, when the target substance M1 is a nucleic acid, the first well W1 in which one target substance M1 is placed may be extracted by performing an ICA reaction in the first well W1.

More specifically, a fluorescent substance different from the fluorescent substance used in step (5) is contained in the sample solution L1 containing nucleic acid, and is then caused to flow through a detection device. Subsequently, in step (2), after placing the target substance M1 in each first well W1, an ICA reaction is performed in the first well W1, and the fluorescence intensity at an arbitrary reaction time is measured, thereby identifying the first well W1 containing one molecule of nucleic acid.

At this time, if the number of light emitting wells is a certain number or less (more specifically, 1/10 of the total number of first wells), it may be regarded that most of the light emitting wells contain only one molecule of nucleic acid based on the Poisson distribution.

After identifying the first well W1 in which one molecule of nucleic acid is placed, the target molecule is detected by step (5).

Further, after visualization by the above method, a micrograph of the first well W1 in which the target substance M1 is placed may be taken, and the taken image may be analyzed to extract the first well W1 in which one target substance M1 is placed.

### [Second embodiment]

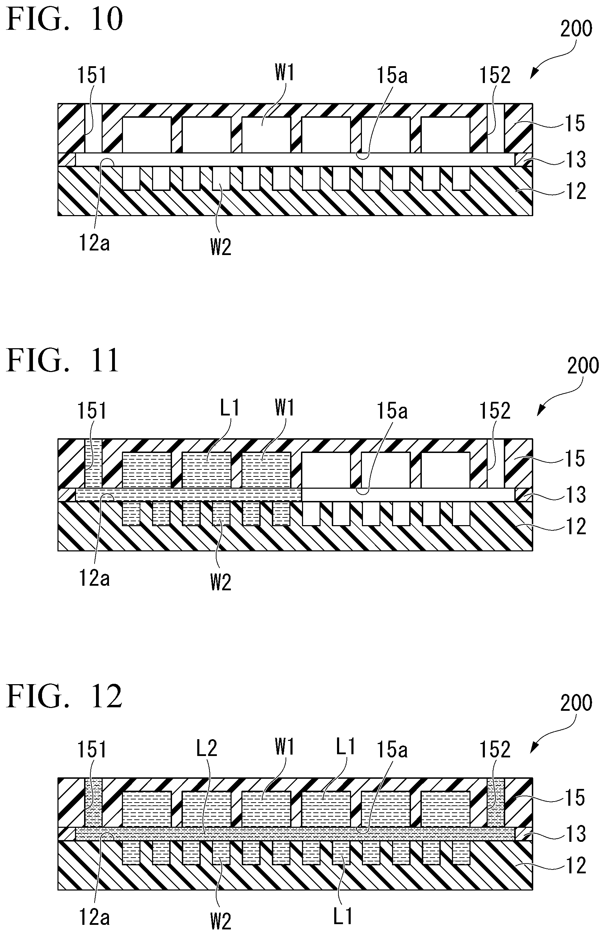

FIGS. 10 to 13 are explanatory diagrams of a detection device and a method for detecting a target molecule according to a second embodiment. Components in the present embodiment that are common to those in the first embodiment are given the same reference numerals, and detailed explanations will be omitted.

### [Detection device]

FIG. 10 is a cross-sectional arrow view showing a detection device 200 according to the present embodiment, and is a diagram corresponding to FIG. 2.

The detection device 200 has a first well plate 15, a second well plate 12, and a wall material 13. The detection device 200 is used as a reaction vessel for accommodating a sample inside, releasing a target molecule from a target substance contained in the sample, and carrying out a detection reaction of the target molecule. At that time, the target molecule is released from the target substance contained in the sample in the first well plate 15, and the target molecule moving from the first well plate 15 is detected in the second well plate 12.

The first well plate 15 and the second well plate 12 are stacked in a freely detachable manner via the wall material 13.

### <First well plate>

The first well plate 15 is a plate-like member that has a rectangular shape in plan view. The first well plate 15 has a plurality of first wells W1 on a well-forming surface 15a that faces the inner surface of the detection device 200. The first wells W1 are recesses provided on the well-forming surface 15a of the first well plate 11 and open to the well-forming surface 15a.

The first well plate 15 has two through holes that penetrate in the thickness direction. The two through holes are provided at one end side and the other end side of the first well plate 15, respectively. One of the through holes is an inlet 151 used when injecting a liquid product into an internal space S of the detection device 200, and the other through hole is an outlet 152 used when discharging a liquid product from the internal space S.

The inlet 151, the internal space S, and the outlet 152 are connected in this order and forming a flow path FC as a whole. In the detection device 200, the liquid product is appropriately caused to flow through the flow path FC to seal the target substance in the first well W1. The first well plate 15 has a plurality of first wells W1 between the inlet 151 and the outlet 152 in plan view.

An upper surface of the first well plate 15 may have a cylindrical injection port surrounding the periphery of the inlet 151. Further, the upper surface of the first well plate 15 may have a cylindrical injection port surrounding the periphery of the outlet 152.

### [Method for detecting target molecule]

FIGS. 11 to 13 are explanatory diagrams of the method for detecting a target molecule in the present embodiment. In the method for detecting a target molecule in the present embodiment, steps (1) to (5) above are performed using the detection device 200. Step (1) is the same as in the first embodiment.

Subsequently, as shown in FIG. 11, a sample solution L1 containing a detection reagent is injected from the inlet 151 into the internal space S (flow path FC), and the first well W1 opening into the internal space S is filled with the sample solution L1. At this time, the second well W2 is also filled with the sample solution L1.

Subsequently, as shown in FIG. 12, a sealing liquid L2 is injected from the inlet 151 into the internal space S (flow path FC). The sealing liquid L2 flows in the internal space S in the surface direction of the well-forming surface 11a, and replaces the internal space S by washing away the sample solution L1 that is not contained in the first well W1 and the second well W2 in the internal space S.

As a result, the sealing liquid L2 seals each of the plurality of first wells W1 and second wells W2 individually (step (2)). The sample solution L1 replaced by the sealing liquid L2 and excess sealing liquid L2 are discharged from the outlet 152.

Subsequently, according to the method described above, the target molecule is released from the target substance M1 distributed to each first well W1 (step (3)).

Then, the first well plate 15 and the second well plate 12 are pressed against each other from both sides of the detection device 200 so as to bring the well-forming surface 15a into contact with the well-forming surface 12a.

As a result, as shown in FIG. 9 in the first embodiment, the first well W1 and the second well W2 are connected, and the target molecule M2 in the first well W1 diffuses and moves to the second well W2 (step (4)).

Then, the target molecule M2 is caused to react with the detection reagent in the second well W2 to detect the target molecule M2 (step (5)).

According to the detection device 200 configured as described above, it becomes possible to detect a target molecule released from a target substance in a solution with high sensitivity.

Further, the target molecule can also be detected with high accuracy according to the method for detecting a target molecule as described above.

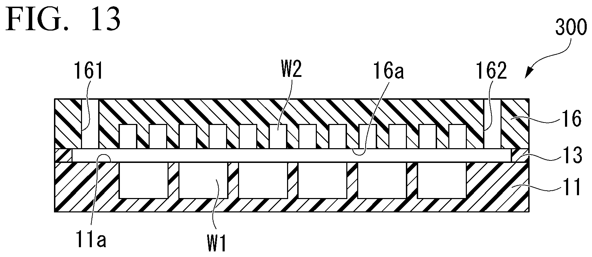

It should be noted that the detection device 200 of the present embodiment is configured to have an inlet 151 and an outlet 152 in the first well plate 15, but is not limited thereto. Like a detection device 300 as shown in FIG. 13, it may be configured to have a first well plate 11 and a second well plate 16 having an inlet 161 and an outlet 162.

The detection device 300 can be used in the same manner as the detection device 200 to carry out the method for detecting a target molecule.

### Examples

The present invention will be described below in more detail with reference to Examples, but the present invention is not limited to the following Examples.

### (Production of detection device)

In this example, a detection device 100 according to the first embodiment described above was used as a detection device. First, a first well plate 11 and a second well plate 12 were produced by injection molding. Similarly, a first lid material 21 and a second lid material 22 were produced by injection molding. A cycloolefin polymer was used as a forming material.

In the first well plate 11, an opening diameter (major axis) of the first well was 50 µm, and a depth of the first well W1 was 50 µm. The volume of each of the first wells W1 was 98 pL.

In the second well plate 12, an opening diameter (major axis) of the second well W2 was 10 µm, and a depth of the second well was 15 µm. The volume of each of the second wells W2 was 1,100 fL.

The first well plate 11 and the first lid material 21 were adhered using a double-sided tape (manufactured by Nitto Denko Corporation) to form a first member 110. Similarly, the second well plate 12 and the second lid material 22 were adhered using a double-sided tape (model number: No. 5610BN, manufactured by Nitto Denko Corporation) to form a second member 120.

### (Preparation of capture particles for capturing cytokines)

A cytokine capture antibody (Anti-Mouse IL-2 MAb (Clone JES6-1A12), model number: MAB702-100, manufactured by R&D Systems, Inc.) was bound to magnetic beads (MS300/Carboxyl, manufactured by JSR Corporation). Furthermore, 100 µL of a buffer (hereinafter, referred to as a buffer B) containing a blocking agent (Pierce Protein-Free (PBS) Blocking Buffer, manufactured by Thermo Fisher Scientific Inc.) was added, and a dispersion liquid containing the magnetic beads to which the cytokine capture antibody had been bound was mixed by inversion at room temperature for 1 hour using a rotator.

The obtained magnetic beads (capture particles) were washed once with 100 µL of PBS-T. The washed magnetic beads were dispersed in a culture medium 2 to prepare a blocking bead solution.

### (Step (1))

Cells were cultured in the following culture medium 1 under conditions of 37°C and 5% CO₂.

Cell: mouse lymphoid cell EL-4 (ATCC TIB39: manufactured by ATCC)

### Culture medium 1:

D-MEM (High Glucose) (manufactured by FUJIFILM Wako Pure Chemical Corporation)
10% by volume FBS, Qualified (gibco, manufactured by Thermo Fisher Scientific Inc.)
1% by volume penicillin-streptomycin solution (manufactured by FUJIFILM Wako Pure Chemical Corporation)

The cell culture medium containing the above cells was removed by a centrifuge operation. A culture medium 2 having the following composition was added to the obtained cell pellet. The cell concentration in the cell culture medium was adjusted to 1.0 × 10⁶ cells/mL.

### Culture medium 2:

D-MEM (High Glucose) (manufactured by FUJIFILM Wako Pure Chemical Corporation)
10% by volume FBS, Qualified (gibco, manufactured by Thermo Fisher Scientific Inc.)
1% penicillin-streptomycin solution (manufactured by FUJIFILM Wako Pure Chemical Corporation)
1% alamarBlue Cell Viability Reagent (manufactured by Thermo Fisher Scientific Inc.)
10 ng/ml Phorbol 12-myristate 13-acetate (mouse skin tumor promoter, manufactured by Adipogen Life Sciences, Inc.)

### (Step (2))

The first member 110 and the second member 120 were respectively filled with pure water. After that, the buffer B was added to the first well W1 of the first member 110.

Then, the blocking bead solution described above was added so that the amount of captured particles added to the first member 110 was 1 × 10⁷ particles.

Subsequently, 20 µL of the prepared cell culture medium and 20 µL of cell-free culture medium were added to the first member 110 (see FIG. 4). The presence or absence of luminescence in each well was confirmed by this operation, and this was used as a control experiment (negative control) for the subsequent operations.

Then, 500 µL of FC-40 was filled into the first member 110, and the cell culture medium was sealed in the first well W1 (see FIG. 5).

### (Step (3))

The first member 110 was placed in an incubator (Mini PORTABLE CO2 INCUBATOR, manufactured by AS ONE Corporation), and the cells were cultured for 2 hours under an environment of 37°C and 5% CO₂. As a result, cytokines were released from the EL-4 cells.

### (Step (4))

After culturing, 200 µL of Tween 20-containing FC-40 was added to the first member 110.

The second member 120 was filled with 100 µL of PBS-T (PBS: manufactured by Takara Bio Inc.; 0.05 vol% Tween 20: manufactured by Sigma-Aldrich Co. LLC., acquired by Merck KGaA) (see FIG. 6). Then, 200 µL of FC-40 was added to the second member 120, and PBS-T was sealed for each second well W2 (see FIG. 7).

The first well plate 11 was removed from the first member 110, and the second well plate 12 was removed from the second member 120, respectively. 100 µL of FC-40 was added to each well-forming surface.

With the well-forming surface 11a of the first well plate 11 facing vertically downward and the well-forming surface 12a of the second well plate 12 facing vertically upward, the well-forming surfaces were brought into contact with each other to form a detection device, and both of the well plates were pressed against each other and pasted together (see FIGS. 8 and 9). It is thought that during this operation, the captured particles move from the first well W1 to the second well W2.

With the first well plate 11 and the second well plate 12 stacked on top of each other, the state of the wells was observed using a microscope equipped with a TexasRed filter, and the first well W1 emitting strong fluorescence was identified. It can be determined that cells are present in the first well W1 where fluorescence is confirmed as described above. At the same time, the position of the second well W2 that planarly overlapped with the first well W1 containing cells was identified.

### (Step (5))

The detection device 100 was divided into the first well plate 11 and the second well plate 12, and FC-40 was added to each well-forming surface. The second lid material 22 was readhered to the second well plate 12 to form the second member 120.

Furthermore, 200 µL of FC-40 was added from an inlet 221 of the second member 120.

The second member 120 was placed on a magnet sheet to fix the capture particles (magnetic beads), and 200 µL of wash buffer was added to the second member 120 to wash the inside of the second well W2. The wash buffer was a PBS solution containing 2% by volume of Tween 20 and 5% by volume of Pierce Protein-Free (PBS) Blocking Buffer.

100 µL of an IL-2 detection antibody (JES6-5H4, model number: M101, manufactured by Invitrogen Corporation) to which alkaline phosphatase was bound was added. An AP binding kit available from Dojindo Laboratories was used.

100 µL of FC-40 was added to the second member 120, and the second well W2 was sealed. Then, the second member 120 was left to stand at room temperature for 1 hour.

The second member 120 was placed on a magnetic sheet, and 200 µL of wash buffer was added to wash the inside of the second well W2.

100 µL of a substrate solution having the following composition was added to the second member 120, and 200 µL of FC-40 was further added to the second member 120.

### Substrate solution:

400 µmol/L FDP (manufactured by AAT Bioquest, Inc.)
1 mol/L DEA (Diethanolamine) (pH 9.5) (manufactured by FUJIFILM Wako Pure Chemical Corporation)
1 mmol/L MgCl2 (manufactured by Thermo Fisher Scientific Inc.)
0.05 vol% Tween 20 (manufactured by Sigma-Aldrich Co. LLC., acquired by Merck KGaA).

Microscopic observation was performed following standing at room temperature for 45 minutes. The following equipment was used.
Microscope: BZ-800 (manufactured by Keyence Corporation)
Objective lens: CFI Plan Apochromat Lambda 10X (manufactured by Nikon Corporation)
Filter 1: BZ-X filter TexasRed (manufactured by Keyence Corporation)
Filter 2: BZ-X filter GFP (manufactured by Keyence Corporation)

As a result of the experiment, it was possible to identify a well in which beads were present and light was emitted, among the second wells W2 that planarly overlapped the first wells W1 containing cells.

When observing 100 first wells W1 in which cells were confirmed, among the second wells W2 that planarly overlapped the first wells W1 containing cells, the number of second wells W2 in which beads were present and light was emitted was 20. The ratio of light-emitting second wells W2 with respect to first wells W1 in which cells were confirmed was 20%.

On the other hand, when observing 736 first wells W1 in which no cells were found, among the second wells W2 that planarly overlapped the first wells W1, the number of second wells W2 in which beads were present and light was emitted was 3. The ratio of light-emitting second wells W2 with respect to first wells W1 in which no cells were found was 0.41% (blank test).

From the above results, it can be determined that the reason the second wells W2 were emitting light was due to cytokines released from cells in the first wells W1, and the usefulness of the present invention was verified.

### INDUSTRIAL APPLICABILITY

According to the present invention, it is possible to provide a detection device that can detect a target molecule released from a target substance in a solution with high sensitivity. In addition, it is possible to provide a method for detecting a target molecule using such a detection device.

### REFERENCE SIGNS LIST

11, 15: First well plate; 12, 16: Second well plate; 13: Wall material; 21: First lid material; 22: Second lid material; 100, 200, 300: Detection device; 110: First member; 120: Second member; 151, 161, 211, 221: Inlet; 152, 162, 212, 222: Outlet; FC, FC1, FC2: Flow path; L1, L2: Sample solution; L2: Sealing liquid; M1: Target substance; M2: Target molecule; W1: First well; W2: Second well

## Claims

1. A detection device comprising:
a first well plate having a plurality of first wells on one surface; and
a second well plate having a plurality of second wells on one surface,
wherein said first well plate and said second well plate are spaced apart from each other and cause said first wells and said second wells to face each other, and
said first wells overlap said second wells in plan view.

2. The detection device according to Claim 1,
wherein an opening diameter of said second wells is smaller than an opening diameter of said first wells, and
said first wells overlap with two or more of said second wells in plan view.

3. The detection device according to Claim 1 or 2, further comprising a wall material which is sandwiched between said first well plate and said second well plate and separates said first well plate from said second well plate.

4. The detection device according to Claim 3, wherein said wall material is integrally formed with either one of said first well plate and said second well plate.

5. The detection device according to Claim 3, wherein said first well plate and said second well plate are stacked in a freely detachable manner via said wall material.

6. The detection device according to Claim 1, further comprising:
a first lid material which is freely detachable from said first well plate and forms a first member; and
a second lid material which is freely detachable from said second well plate and forms a second member,
wherein said first lid material has an inlet and an outlet penetrating in a thickness direction,
a flow path through which a fluid flows is formed in said first member between said first well plate and said first lid material,
said second lid material has an inlet and an outlet penetrating in a thickness direction, and
a flow path through which a fluid flows is formed in said second member between said second well plate and said second lid material.

7. The detection device according to Claim 1, wherein a major axis of an opening of said first well is 1 µm or more and 1 cm or less.

8. The detection device according to Claim 1, wherein an aspect ratio of said first well is 0.002 or more and 3 or less.

9. The detection device according to Claim 1, wherein a major axis of an opening of said second well is 1 nm or more and 1 mm or less.

10. The detection device according to Claim 1, wherein an aspect ratio of said second well is 0.05 or more and 3 or less.

11. The detection device according to Claim 1, wherein said first well overlaps with 1 to 10,000 of said second wells in plan view.

12. The detection device according to Claim 1,
wherein a ratio of a volume of one of said first wells to a volume of one of said second wells ([first well]: [second well]) is from 500,000: 1 to 1: 1.

13. A method for detecting a target molecule using the detection device according to Claim 1, the method comprising:
a step of filling a sample solution containing a target substance into each of said plurality of first wells independently;
a step of releasing said target molecule from said target substance in one of the first wells in which said target substance is placed among a plurality of said first wells;
a step of bringing a surface onto which said first well is formed in said first well plate into contact with a surface onto which said second well is formed in said second well plate to distribute said target molecule one molecule at a time to each of said second wells independent of each other from said first well; and
a step of causing a reaction between said target molecule and a detection reagent and detecting said target molecule.

14. The method for detecting a target molecule according to Claim 13, wherein in said filling step, said sample solution is sealed in said first well with a sealing liquid.

15. The method for detecting a target molecule according to Claim 14, wherein said sealing liquid is a fluorine-based oil.

16. The method for detecting a target molecule according to Claim 13, wherein said sample solution contains a surfactant.

17. The method for detecting a target molecule according to Claim 13, further comprising, prior to said filling step,
a step of obtaining said sample solution adjusted to a concentration such that one of said target substances is placed in each of said first wells based on a volume of said detection device.
